# EUROPÄISCHE PATENTANMELDUNG

(11) **EP 0 639 551 A1**
(43) Veröffentlichungstag der Anmeldung: **22.02.1995**
(21) Anmeldenummer: 94109268.6
(22) Anmeldetag: 16.06.1994
(51) Int. Cl.: C07C 29/86, C07H 15/04, C07C 31/125

(54) **Entfernung von höheren Alkoholen von Alkylpolyglycosiden**

(30) Priorität: 20.08.1993 DE 4328008
(71) Anmelder: HÜLS AKTIENGESELLSCHAFT, D-45764 Marl (DE)
(72) Erfinder: Sridhar, Srinivasan, Dr., D-45770 Marl (DE); Müller, Wolfgang Hans Eduard, Dr., D-45768 Marl (DE)

(57) **Zusammenfassung**

Die Erfindung betrifft die Extraktion von höheren Alkoholen aus einem Gemisch dieser höheren Alkohole und Alkylpolyglycosiden mit Ethan im überkritischen Zustand als Extraktionsmittel.

Ethan erlaubt höhere Beladungen und wesentlich kürzere Extraktionszeiten als das bisher üblicherweise eingesetzte überkritische Kohlendioxid.

## Beschreibung

Die Erfindung betrifft ein Verfahren zur Abtrennung von Alkoholen aus einem Gemisch dieser Alkohole und Alkylpolyglycosiden unter Verwendung von Ethan im überkritischen Zustand als Extraktionsmittel.

Alkylpolyglycoside (APG) können durch Umsetzung von Alkoholen mit Mono-, Di- oder Oligosacchariden, die als Bausteine Pentosen bzw. Hexosen wie z. B. Glucose enthalten, hergestellt werden. In der Direktsynthese erfolgt im allgemeinen eine unmittelbare Umsetzung der Saccharide mit langkettigen Alkoholen mit 8 bis 20 Kohlenstoff-Atomen je Molekül. Bei der indirekten Synthese werden die Saccharide in einem ersten Schritt mit einem Alkohol mit weniger als 8 Kohlenstoffatomen wie z. B. Butanol, umgesetzt und die dadurch gewonnenen niederen Alkylglycoside mit längerkettigen Alkoholen umglycosidiert. Die jeweilige Glycosidierung kann unter Einsatz eines Säurekatalysators, beispielsweise p-Toluolsulfonsäure, erfolgen. Da man bei der Glycosidierung bzw. Umglycosidierung einen großen Überschuß an Alkoholen, die nicht nur als Reaktant sondern auch als Lösungsmittel fungieren, vorlegen muß, hat man nach der Reaktion ein Gemisch vorliegen, das das gewünschte Produkt APG und etwa 70 - 95 Gew.-% Alkohol enthält. Die Alkohole müssen vom Produkt abgetrennt werden.

Die Abtrennung der Alkohole kann thermisch, beispielsweise durch Einsatz eines Dünnschichtverdampfers, erfolgen. Da jedoch die höheren Alkohole auch im besten Vakuum (1 hPa) erst bei Temperaturen oberhalb 150 °C sieden, ist eine thermische Belastung des Produktes unvermeidlich. Dieser Umstand führt zu einer Produktschädigung, die sich durch eine Dunkelfärbung bzw. Verfärbung des Produktes bemerkbar macht. Da die APG mit langkettigen Alkylresten u. a. ihre Verwendung in Naschmitteln finden, müssen sie einen umfangreichen Bleichprozeß durchlaufen, bevor sie in den Markt gelangen.

In der Literatur wird auf die Möglichkeit einer Alkoholabtrennung mittels überkritischer Gase hingewiesen. JP 91/027 336 A, JP 92/193 304 A und JP 90/184 388 A beschreiben die Entwässerung von Alkoholen (vermutlich Ethanol) mittels überkritischer Gase wie Propan, Propylen und Butan. JP 89/083 633 A beschreibt die Abtrennung höherer Alkohole aus kurzkettigen Fasern auch mittels Ethylen und Ethan neben Kohlendioxid, aber hier wird Kohlendioxid als das bevorzugte Mittel genannt. Ebenfalls wird in JP 87/087 537 A Kohlendioxid als Extraktionsmittel für C₂₈/C₃₀-Alkohole genannt. Weiterhin wird ein Verfahren beschrieben (JP 90/103 202 A), bei dem Kohlendioxid im überkritischen Zustand als Extraktionsmittel für Alkohole aus langkettigen Alkylpolysacchariden angewendet wird. Damit werden die Alkohole des Gemisches im Gas gelöst und abgeführt, während die Alkylpolysaccharide als Rückstand bleiben. Als weitere überkritische Gase werden ausdrücklich auch Ethylen, Propan und Propylen, nicht jedoch Ethan, erwähnt. Die Beispiele beziehen sich jedoch ausschließlich auf die Verwendung von Kohlendioxid, und dieses Gas wird als sinnvoll, geeignet und ausreichend dargestellt. Bei 45 °C und ca. 20 MPa wird das Gemisch unter Umrühren mit Kohlendioxid behandelt. In einem weiteren Verfahren (JP 91/250 001 A) wird bei Beibehaltung des Gases dem Ausgangsgemisch zusätzlich ein Gemisch aus Ethanol und Wasser zugeführt, das die Aufgabe hat, das Ausgangsgemisch flüssig zu halten, damit die Extraktion in einer Kolonne erfolgen kann. Nach wie vor ist auch hier ein hoher Druck von ca. 25 MPa erforderlich. Trotz der Maßnahme der Extraktion in einer Kolonne bei erhöhtem Stoffübergang und bei erhöhtem Druck sind 125 kg CO₂/kg Einsatzgemisch erforderlich.

Nach allen diesen Verfahren wird daher Kohlendioxid als das bevorzugte Mittel für die überkritische Extraktion von Alkoholen angesehen.

Bei einer Überprüfung der obengenannten Lehre stellte sich jedoch heraus, daß Kohlendioxid zwar prinzipiell eine extraktive Wirkung aufweist, aber ein schlechtes Aufnahmevermögen für die Alkohole hat. Trotz einer Vorstufe der Extraktion im Gegenstrom und einer Reduzierung des Alkoholgehaltes von ca. 80 auf 40 - 60 Gew.-% Alkohol im Gemisch reichte eine weitere Extraktion des fast festen Gemisches über eine Dauer von 18 bis 24 h noch nicht aus, um den Alkoholgehalt im Rückstand (APG) auf < 2 Gew.-% zu senken. Erst bei noch wesentlich längeren Extraktionszeiten gelang es, den Alkoholgehalt im Rückstand auf den geforderten Wert von < 2 Gew.-% abzusenken.

Die Aufgabe der Erfindung bestand deshalb darin, ein Verfahren zur Abtrennung von Alkoholen aus APG unter Verwendung eines leistungsfähigeren überkritischen Gases als Extraktionsmittel zu finden, das höhere Beladungen und wesentlich kürzere Extraktionszeiten mit den damit verbundenen Vorteilen der Material- und Energieersparnis zur Erreichung des aus anwendungstechnischen Gründen geforderten Grenzwertes von < 2 Gew.-% Alkohol erlaubt.

Die Aufgabe der Erfindung wird dadurch gelöst, daß im Verfahren Ethan im überkritischen Zustand als Extraktionsmittel verwendet wird.

Gegenstand der Erfindung ist daher ein Verfahren zur Abtrennung von Alkoholen aus einem Gemisch dieser Alkohole und Alkylpolyglycosiden, dadurch gekennzeichnet, daß die Abtrennung unter Verwendung von Ethan im überkritischen Zustand als Extraktionsmittel erfolgt.

Das oben erwähnte Gemisch aus APG und Alkoholen mit 8 bis 20, vorzugsweise 8 bis 18 und besonders bevorzugt 10 bis 16, C-Atomen wird demgemäß bei oder oberhalb der kritischen Temperatur des Ethans (32,4 °C), und bei Drücken bei oder oberhalb des kritischen Drucks von Ethan (4,9 MPa), in Berührung gebracht. Auf Maßnahmen, wie eine Einführung von Wasser und von systemfremden Stoffen, kann verzichtet werden. Vorteilhaft ist eine möglichst niedrige Temperatur etwas oberhalb der kritischen Temperatur von Ethan, bei der die Gasdichte am höchsten ist. Eine allzu hohe Temperatur weit entfernt von der kritischen Temperatur von Ethan ist aus Gründen der thermischen Belastung zu vermeiden. Die Extraktion wird daher bei Temperaturen im Bereich von der kritischen Temperatur des Ethans (32,4 °C) bis 120 °C durchgeführt. Eine Temperatur im Bereich von 40 - 80 °C wird bevorzugt. Als Druck kann ein beliebig hoher Druck oberhalb des obengenannten kritischen Drucks von Ethan gewählt werden, da in der Regel ein hoher Druck auch die Löslichkeit der Alkohole erhöht. Der Druck kann bis 40 MPa betragen. Da aber sehr hohe Drücke, wie etwa 20 - 25 MPa und höher, die bei den bereits genannten Verfahren mit Kohlendioxid erforderlich sind, unwirtschaftlich sind, werden Drucke im Bereich von 10 - 20 MPa bevorzugt. Die Beispiele zeigen, daß diese Drucke vollkommen ausreichend sind. Bei ihnen wurden 140 - 190 kg Ethan/kg Einsatzgemisch aufgewendet.

Die überkritische Gasphase mit den darin gelösten Alkoholen wird aus dem System abgeführt und die Alkohole durch Druckminderung und eventuelle Temperaturänderung der Gasphase abgetrennt und als Flüssigkeit bzw. Feststoff wiedergewonnen. Der Druck bei der Entspannung und Abtrennung der Gasphase kann sowohl oberhalb als auch bei und unterhalb des kritischen Druckes zwischen 0,1 und 40 MPa, vorzugsweise zwischen 0,5 und 20 MPa, liegen. Die Abtrennung kann sowohl oberhalb als auch bei und unterhalb der kritischen Temperatur erfolgen. Die Temperatur bei der Entspannung liegt vorzugsweise im Bereich von 20 bis 150 °C. Eine Temperatur von 30 - 120 °C wird besonders bevorzugt. Die Bedingungen richten sich nach den apparativen Möglichkeiten und wirtschaftlichen Gegebenheiten. Das von den Alkoholen befreite Gas wird durch Druckerhöhung und Anpassung der Temperatur wieder in den überkritischen Zustand gebracht und in den Extraktionsschritt zurückgeführt.

Für die praktische Durchführung können für Lösungsmittelextraktion bekannte Vorrichtungen wie Extraktionsbehälter oder Kolonnen mit Böden oder Füllkörpern bzw. Gewebepackungen verwendet werden. Für die Auswahl der Apparaturen ist der Aggregatzustand des Gemisches maßgeblich. Sofern das Gemisch (bedingt durch die Art bzw. den Gehalt an Alkohol) fließfähig ist, kann die Extraktion wahlweise in einer Kolonne im Gleich- oder Gegenstrom erfolgen. Bei Gemischen, die zäh und nicht pumpfähig bzw. fest sind, kann die Extraktion stationär in einem Extraktionsbehälter vorgenommen werden. Die Extraktion kann auch ohne Umrühren des Gemisches erfolgen.

### Vergleichsbeispiele

### Beispiel 1:

Überkritisches Kohlendioxid wurde als Extraktionsmittel angewendet. Die Extraktion erfolgte bei 60 °C und 18 MPa und in einer ersten Stufe in einer Kolonne (1m lang, NW 38 mm) mit Gewebepackung der Fa. Sulzer. Das Einsatzgemisch wurde auf die Extraktionstemperatur vorgewärmt und mittels einer Pumpe von oben nach unten durch die Kolonne geleitet, während das überkritische Gas im Gegenstrom von unten nach oben geführt wurde. Es wurden 1 103 g eines Gemisches mit 82,7 Gew.-% C₁₂/C₁₄-Alkoholen und 17,3 Gew.-% Alkylpolyglucosiden bei 1,03 l/h durchströmt. Der Gasstrom entsprach 6,3 kg/h. Das Gas wurde bei 30 °C auf 5 MPa in einem Abscheidebehälter (Separator) vom Rauminhalt 1 l entspannt und im überkritischen Zustand kontinuierlich wieder der Kolonne zugeführt. Das Gemisch wurde nach Austritt aus der Kolonne in einen Vorratsbehälter geführt und ebenfalls kontinuierlich in die Kolonne gefahren. Die Extraktion wurde nach 155 Min. abgebrochen. In einem zweiten Ansatz wurden wieder 1102 g des gleichen Gemisches bei gleichen Bedingungen 160 Minuten lang behandelt. Die vereinigten Rückstände beider Ansätze wiesen noch 56,7 Gew.-% Alkohol auf.

604 g der vereinigten Rückstände, die noch zähflüssig aber kaum noch pumpfähig waren, wurden im Sumpfteil der Kolonne vorgelegt und stationär im Gasstrom bei sonst gleichen Bedingungen und ohne Umrühren weitere 1430 Min. extrahiert. Der endgültige Rückstand wies immer noch 5,9 Gew.-% Alkohol auf. Die gesamte Extraktionsdauer beider Schritte (unter Berücksichtigung des ersten parallel verlaufenen Schrittes) betrug 26,5 h. Im 1. bzw. 2 Schritt wurden 15 bzw. 250 kg CO₂/kg jeweiligen Einsatzgemisches aufgewendet. Der Separatorinhalt bestand zu > 96 % aus Fettalkoholen neben < 4 % Glucosiden.

### Beispiel 2:

Bei gleichen Bedingungen wie im Beispiel 1 wurden wieder zwei Ansätze von 1 048 bzw. 1 105 g eines Gemisches mit 78,9 Gew.-% C₁₂/C₁₄-Alkoholen und 21,1 Gew.-% Alkylpolyglucosiden mit Kohlendioxid extrahiert. Nach 165 bzw. 175 Min. wurde ein Alkoholgehalt von 44,1 Gew.-% in den vereinigten Rückständen erreicht. Eine weitere stationäre Extraktion von 464 g der vereinigten Rückstände wie im Beispiel 1 führte nach 690 Min. in diesem Schritt (Gesamtdauer einschließlich des 1. Schrittes = 14,3 h) bzw. nach 1075 Min. in diesem Schritt (Gesamtdauer 20,8 h) zum Restgehalt von 7,8 bzw. 5,3 Gew.-% an Alkohol im Rückstand. Im 1. bzw. 2. Schritt bis 1 075 Min. wurden 16 bzw. 243 kg CO₂/kg jeweiligen Einsatzgemisches aufgewendet.

### Erfindungsgemäße Beispiele

### Beispiel 3:

108 g des verbleibenden Rückstands nach dem Beispiel 1 wurden stationär und ohne Umrühren in einem Extraktionsbehälter vom Rauminhalt 2,5 l bei 25 MPa und 60 °C mittels 15 kg/h Ethan 1065 Min. behandelt. Bei der Entspannung herrschten die Bedingungen 30 °C und 6 MPa. Es wurde ein niedriger Wert von nur noch 0,8 Gew.-% Restgehalt an Alkohol im Rückstand erreicht.

### Beispiel 4:

126,4 g eines Gemisches aus Alkylpolyglucosiden und C₁₂/C₁₄-Alkoholen, das 82 Gew.-% Alkohol und 18 Gew.-% Alkylpolyglucosid enthielt, wurde in einem Extraktionsbehälter wie im Beispiel 3 bei 50 °C und einem niedrigeren Druck von 13 MPa mit einem Ethanstrom von 8,9 kg/h 130 Min. lang versetzt. Auf ein Umrühren wurde verzichtet. Der Ethanstrom wurde laufend aus dem Behälter abgeführt und im Separator bei 30 °C auf 3 MPa entspannt und von den Alkoholen befreit. Nach 130 Min. waren im Alkylpolyglucosid-Rückstand nur noch 1,9 Gew-% Alkohol enthalten. Es wurden 150 kg Ethan/kg Einsatzmenge verwendet. Der abgeschiedene Inhalt im Separator bestand zu 96 Gew-% aus den Alkoholen und 4 Gew.-% aus Glucosiden.

### Beispiel 5:

125,6 g eines Gemisches mit 76 Gew.-% C₁₂/C₁₄-Fettalkoholen und 24 Gew.-% Alkylpolyglucosiden wurden gemäß Beispiel 4 aber bei einer Extraktionstemperatur von 80 °C 160 Min. lang behandelt. Es wurde ein Rückstand mit 1,7 Gew.-% Restgehalt an Alkohol erzielt. Es wurden 189 kg Ethan/kg Einsatzmenge verwendet.

### Beispiel 6:

128,3 g eines Gemisches wie im Beispiel 4, aber mit 87 Gew.-% C₁₆/C₁₈-Alkoholen und 13 Gew.-% Alkylpolyglucosiden wurden ebenfalls bei gleichen Bedingungen 120 Min. lang behandelt. Der Restgehalt an Alkohol im Rückstand betrug 1,1 Gew.-%. Es wurden 139 kg Ethan/kg Einsatzmenge verwendet.

### Beispiel 7:

125 g desselben Gemisches wie im Beispiel 6 wurden 120 Minuten lang gemäß Beispiel 1 behandelt. Die Temperatur im Separator lag aber bei 50 °C. Der Restgehalt an Alkohol im Rückstand zum Versuchsende betrug nur noch 1,2 Gew.-%. Es wurden 142 kg Ethan/kg Einsatzmenge verwendet.

### Beispiel 8:

251 g eines Gemisches aus 93 Gew.-% C₁₆/C₁₈-Alkoholen und 7 Gew.-% Alkylpolyglucosiden wurden 300 Min. lang gemäß Beispiel 4 extraktiv behandelt. Der Rückstand enthielt nur noch 1,3 Gew.-% Fettalkohol. Es wurden 177 kg Ethan/kg Einsatzmenge verwendet.

## Patentansprüche

1. Verfahren zur Abtrennung von Alkoholen aus einem Gemisch dieser Alkohole und Alkylpolyglycosiden,
dadurch gekennzeichnet,
daß die Abtrennung unter Verwendung von Ethan im überkritischen Zustand als Extraktionsmittel erfolgt.

2. Verfahren nach Anspruch 1,
dadurch gekennzeichnet,
daß Alkohole mit 8 bis 20, vorzugsweise 8 bis 18 und besonders bevorzugt 10 bis 16, C-Atomen je Molekül von den Alkylpolyglycosiden abgetrennt werden.

3. Verfahren nach den Ansprüchen 1 und 2,
dadurch gekennzeichnet,
daß die Extraktion bei Temperaturen im Bereich von der kritischen Temperatur des Ethans (32,4 °C) bis 120 °C, vorzugsweise 40 bis 80 °C, durchgeführt wird.

4. Verfahren nach den Ansprüchen 1 bis 3,
dadurch gekennzeichnet,
daß die Extraktion bei Drücken im Bereich von dem kritischen Druck des Ethans (4,9 MPa) bis 40 MPa, vorzugsweise 10 bis 20 MPa, durchgeführt wird.

5. Verfahren nach den Ansprüchen 1 bis 4,
dadurch gekennzeichnet,
daß die Extraktion zum Teil oder im Ganzen in einer Extraktionskolonne unter Führung der Gas- und Produktströme im Gleichstrom durchgeführt wird.

6. Verfahren nach den Ansprüchen 1 bis 4,
dadurch gekennzeichnet,
daß die Extraktion zum Teil oder im Ganzen in einer Extraktionskolonne unter Führung der Gas- und Produktströme im Gegenstrom durchgeführt wird.

7. Verfahren nach den Ansprüchen 1 bis 4,
dadurch gekennzeichnet,
daß die Extraktion in einem Extraktionsbehälter stationär ohne Umrühren des Gemisches durchgeführt wird.

8. Verfahren nach den Ansprüchen 1 bis 7,
dadurch gekennzeichnet,
daß das Extraktionsmittel durch Entspannen in einem Abscheidebehälter von den gelösten Alkoholen befreit wird.

9. Verfahren nach den Ansprüchen 1 bis 8,
dadurch gekennzeichnet,
daß Alkylpolyglycoside mit einem Restgehalt von < 2 Gew.-% Alkohole erzielt werden.

10. Verfahren nach den Ansprüchen 1 bis 9,
dadurch gekennzeichnet,
daß das Alkylpolyglycosid ein Alkylpolyglucosid ist.

11. Verwendung von Ethan im überkritischen Zustand zur Extraktion von Alkoholen aus Alkylpolyglykosiden.
